(19) Europäisches Patentamt
European Patent Office
Office européen des brevets

(11) Numéro de publication: **0 068 978 B1**

# (12) FASCICULE DE BREVET EUROPEEN

(45) Date de publication du fascicule du brevet: **03.09.86**

(21) Numéro de dépôt: **82401095.3**

(22) Date de dépôt: **16.06.82**

(51) Int. Cl.⁴: **C 07 D 333/20,** C 07 D 409/12, C 07 D 333/32 // C07D333/22, C07F9/40

## (54) Procédé de préparation de dérivés des (thiényl-2)- et (thiényl-3)-2 éthylamines, et produits ainsi obtenus.

(30) Priorité: **30.06.81 FR 8113062**

(43) Date de publication de la demande:
**05.01.83 Bulletin 83/01**

(45) Mention de la délivrance du brevet:
**03.09.86 Bulletin 86/36**

(84) Etats contractants désignés:
**AT BE CH DE FR GB IT LI LU NL SE**

(58) Documents cités:
**CA-A-1 088 539**
**FR-A-2 300 090**

**JUSTUS LIEBIGS: "Annalen der Chemie", vol. 686, 1965, pages 107-114, Verlag Chemie GmbH, Weinheim, DE. H. ZIMMER et al.: "Synthesen mit Alpha-Amino-Alkyl-Phosphonsäureestern, 1"**

**Le dossier contient des informations techniques présentées postérieurement au dépôt de la demande et ne figurant pas dans le présent fascicule.**

(73) Titulaire: **SANOFI, Société dite:**
**40, Avenue George V**
**F-75008 Paris (FR)**

(72) Inventeur: **Chekroun, Isaac**
**26 Rue Peyras**
**F-31000 Toulouse (FR)**
Inventeur: **Heymes, Alain**
**Chemin de l'Adrech**
**F-04200 Sisteron (FR)**

(74) Mandataire: **Bressand, Georges et al**
**c/o CABINET LAVOIX 2 Place d'Estienne d'Orves**
**F-75441 Paris Cedex 09 (FR)**

(56) Documents cités:
**TETRAHEDRON LETTERS, no. 26, 1979, pages 2433-2436, Pergamon Press Ltd., G.B. N.L.J.M. BROEKHOF et al.: "Enamine synthesis by the Horner-Wittig reaction"**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 068 978 B1

Courier Press, Leamington Spa, England.

**Déscription**

La présente invention est relative à un nouveau procédé de préparation de thiénylamines répondant à formule I

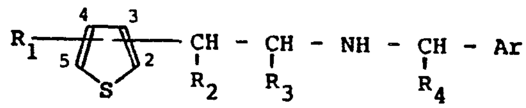

(I)

dans laquelle:

$R_1$ (en position 2, 3, 4 ou 5) représente un atome d'hydrogène ou d'halogène, un radical nitro, carboxyle, cyano, amino, un radical alkyle ou alcoxy linéaire ou ramifié,

dans la chaine aminoéthyle qui peut occuper les positions 2 ou 3 du noyau thiophénique, $R_2$, $R_3$, $R_4$ représentent un atome d'hydrogène ou un radial alkyle linéaire ou ramifié, ou phényle,

Ar représente un radical thiényle, furyle, pyridyle ou phényle, éventuellement mono- ou polysubstitué par des groupes halogéno ou nitro.

Un certain nombre de dérivés répondant à la formule I sont connus et utilisés comme intermédiaires dans la préparation de composés employés aussi bien dans l'industrie chimique que pharmaceutique.

C'est ainsi, qu'à titre d'exemple, on peut mentionner parmi les dérivés obtenus selon le nouveau procédé de préparation ceux qui peuvent conduire par des moyens connus, d'une part (la chaine aminoéthyle étant en 2 et le radical $R_1$ en 4 ou 5) aux dérivés de la tétrahydro 4,5,6,7 thiéno [3,2-C] pyridine, d'autre part (la chaine aminoéthyle étant en 3 et le radical $R_1$ en 4 ou 5) à ceux de la tétrahydro 4,5,6,7 thiéno [2,3-C] pyridine, dérivés qui dans les deux cas ont fait l'objet de la part de la demanderesse pour leurs applications en thérapeutique et/ou leurs procédés de préparation de plusieurs brevets (FR. 2 215 948, FR. 2 300 090, FR. 2 315 274, FR. 2 319 642, FR. 2 345 150, FR. 2 336 932, FR. 2 397 417, ainsi que CA—A—1 088 539).

Par ailleurs, on connaît la réaction de synthèse de Horner-Wittig dans laquelle on fait réagir des composés carbonylés sur des esters d'acides alpha-aminoalkyl-phosphoniques, par exemple selon H. ZIMMER et J. P. BERCZ dans JUSTUS LIEBIGS "Annalen der Chemie", vol. 686, 1965, p. 107—114 ainsi que N:L J. M. BROEKHOF et coll. dans TETRAHEDRON LETTERS, n° 26, 1979, p. 2433—6.

L'invention a pour objet un procédé simple et peu coûteux, en regard ce le technique antérieure, d'obtention des composés de formule I.

Conformément au procédé de l'invention, les différentes étapes sont les suivantes:

a) un dérivé de formule II:

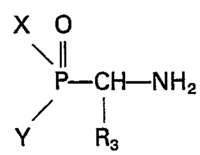

(II)

dans laquelle $R_3$ est tel que défini dans la formule I, X et Y représentent ensemble ou séparément, un radical aryle ou alcoxy de telle sorte que le composé organophosphoré de formule II peut être par exemple un phosphonate, un phosphinate ou un oxyde de phosphine, est condensé avec un composé carbonylé de formule III:

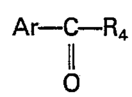

(III)

dans laquelle Ar et $R_4$ sont tels que définis pour la formule I, pour obtenir un dérivé de formule IV:

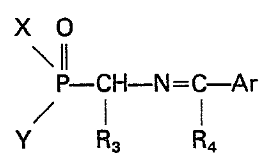

(IV)

dans laquelle X, Y, $R_3$, $R_4$ et Ar sont tels que déffinis pour les formules I et II,

b) le composé de formule IV est traité avec une base de formule $B^{\ominus}$ $M^{\oplus}$ pour conduire à un carbanion de formule V.

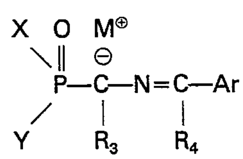

(V)

Cette réaction est conduite à une température comprise entre −78°C et +100°C choisie, plus spécifiquement en fonction de la base $B^{\ominus}$ $M^{\oplus}$ dans une plage plutôt bas de gamme en vue d'éviter d'aboutir à un dérivé de structure VI.

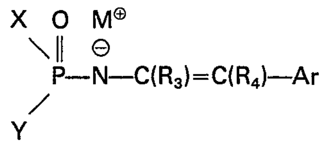

(VI)

c) le carbanion de formule V est ensuite mis à réagir avec un dérivé carbonylé de formule VII:

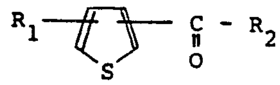

(VII)

dans laquelle $R_1$ et $R_2$ sont tels que définis pour la formule I pour conduire à un composé de formule VIII.

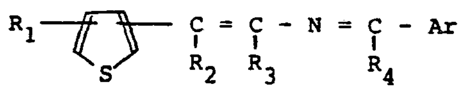

(VIII)

dans laquelle les différents radicaux ont la signification fournie précédemment.

d) le dérivé de formule VIII sous l'action d'un agent réducteur tel que notamment un borohydrure de métal alcalin, est finalement transformé en composé de formule I tel que défini plus haut.

Le procédé de préparation suivant l'invention peut être illustré par le schéma réactionnel suivant:

a. 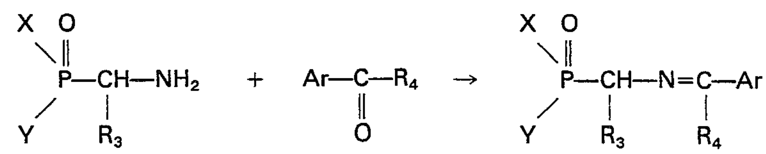

(II) (III) (IV)

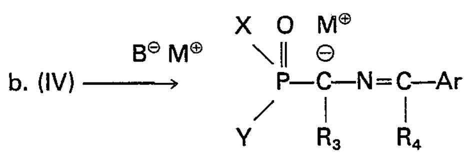


(V)

c. 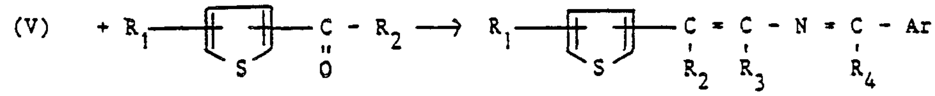

(VII) (VIII)

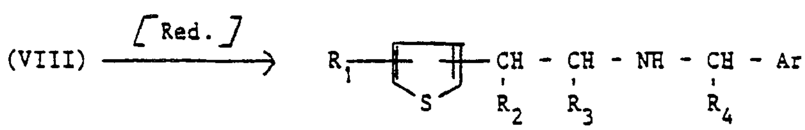


(I)

La procédé peut avantageusement être mis en oeuvre comme suite:

a) Les composés organophosphorés de formule II, facilement accessibles par des procédés d'obtention bien connus tel que celui par exemple décrit par I. C. POPOFF et Coll. (J. Org. Chem. *28*, 2898 (1963)) peuvent être mis à réagir avec les dérivés carbonylés III en l'absence de solvant et de catalyseur, l'eau formée au cours de la réaction étant éliminée à la fin de l'opération par des moyens appropriés. La condensation peut s'effectuer avantageusement dans un solvant tel qu'un hydrocarbure aromatique par exemple le toluène ou un alcool par exemple l'éthanol dans lesquels il est possible d'éliminer l'eau par distillation azéotropique. La condensation peut encore être avantageusement réalisée (sur le plan de la vitesse) en présence de quantités catalytiques d'un acide minéral ou organique comme par exemple l'acide paratoluènesulfonique. La température à laquelle on effectue cette transformation est variable mais se situe très généralement entre 20 et 120°C,

b) La base $B^{\ominus}$ $M^{\oplus}$ mise en oeuvre peut être un hydrure de métal alcalin, notamment les hydrures de sodium, de lithium ou de potassium, un amidure ou alcoylamidure, notamment dialcoylamidure, de métal alcalin tel que la diisopropylamidure de lithium, un composé organométallique, notamment les organolithiens tels que le n-butyllithium ou les organosodés ou les organomangésiens. On peut aussi faire appel aux alcoolates de métal alcalin ou alcalino-terreux, tels que le méthylate de sodium, de lithium, de potassium, de magnésium, le tertiobutylate de potassium, le tertioamylate de sodium. On peut encore utiliser les hydroxydes de métal alcalin ou alcalino-terreux, tels que l'hydroxyde de sodium, de lithium, de potassium, de magnésium.

En général, on utilise la base en léger excès, par exemple de 10% par rapport à l'équivalence stoechiométrique.

On opère généralement entre −78°C et +100°C, à une température choisie plus spécifiquement en fonction de la base $B^{\ominus}$ $M^{\oplus}$ dans une plage plutôt bas de gamme afin d'éviter, ainsi que déjà mentionné, la formation du dérivé VI.

Les solvants préférés sont les éthers linéaires ou cycliques tel que le tétrahydrofuranne, les hydrocarbures, notamment les aromatiques tels que benzène, toluène, xylènes, les alcools, les amides notamment le diméthylformamide, les sulfoxides notamment le diméthylsulfoxide. Il peut être également avantageux, particulièrement quand on utilise des hydroxydes métalliques, d'opérer en système biphasique (Eau + solvant tel que solvant halogéné comme par exemple le dichlorométhane, ou hydrocarbure aromatique tels que benzène, toluène, xylènes) en présence d'un catalyseur de transfert de phase notamment un sel d'ammonium quaternaire tel que l'iodure de tétra-n-buthyl-ammonium ou un sel de phosphonium.

c) Le dérivé V est mis à réagir avec le composé carbonylé de formule VII dans le milieu réactionnel tel que défini ci-dessus à une température comprise entre −78°C et +100°C et choisie plus spécifiquement en fonction de la base $B^{\ominus}$ $M^{\oplus}$ dans un plage plutôt bas de gamme pour les raisons déjà mentionnées.

d) La réduction du dérivé de formule VIII est effectuée avantageusement par un hydrure mixte de métal alcalin comme notamment un borohydrure tel que le borohydrure de sodium ou le borohydrure de potassium. La réduction est réalisée au sein d'un solvant inerte comme un éther tel que par exemple le tétrahydrofurane ou le dioxanne ou encore dans un alcool tel que par exemple le méthanol ou l'éthanol. Il peut être avantageux dans certains cas, et plus particulièrement dans le cas ou au moins l'un des radicaux $R_2$, $R_3$, $R_4$ n'est pas un atome d'hydrogène, d'additionner au milieu réactionnel un équivalent molaire par rapport au borohydrure mis en oeuvre, d'un acide organique, comme par exemple l'acide acétique ou l'acide trifluoracétique.

On peut également effectuer cette réduction par lee biais d'une hydrogénation catalytique en phase homogène ou hétérogène dans des conditions bien connues d'une manière générale.

Les composés de formule I ainsi obtenus peuvent ensuite être isolés et purifiés selon les méthodes habituelles. Pour réaliser ces opérations, il peut être avantageux de transformer les composés libres de formule I en leurs sels, par exemple en leurs sels d'addition d'acides par réaction avec des acides minéraux ou organiques. A partir des sels, on peut libérer les composés de formule I selon les méthodes connues.

Les exemples suivants illustrent l'invention sans la limiter.

Exemple 1
Preparation du chlorhydrate de la N-orthochlorobenzyl, (thienyl-2)-2 ethylamine

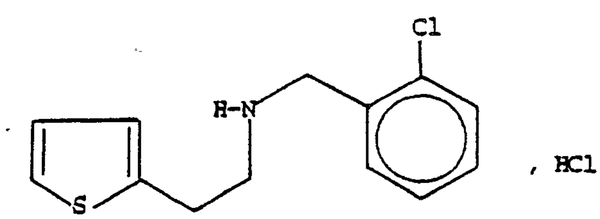

*Stade a. N-orthochlorobenzylidène, aminométhylphosphonate de diéthyle*

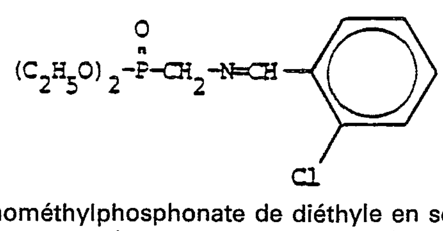

A 16,7 g (0,1 mole) d'aminométhylphosphonate de diéthyle en solution dans 200 ml de toluène, on ajoute, à température ambiante, goutte à goutte, 14 g (0,1 mole) de chloro-2 benzaldéhyde. A la fin de l'addition, on poursuit l'agitation pendant 30'. L'eau formée au cours de la réaction est éliminée par décantation. La phase toluénique est lavée avec 50 ml d'une solution aqueuse saturée de chlorure de

sodium, séchée sur sulfate de sodium puis évaporée. On obtient 29 g (rendement 100%) de N-orthochlorobenzylidène aminométhylphosphonate de diéthyle, sous forme d'une huile jaune monotache en CCM (plaque de silice, éluant AcOEt, rf = 0,45).

IR (film):
- C=N 1635 $cm^{-1}$
- P=O 1250 $cm^{-1}$
- P—O—C 1060 $cm^{-1}$—1030 $cm^{-1}$

RMN ($CDCl_3$) δ/TMS:
- 1,35 ppm (t, SH)
- 4,2 ppm (m, 6H)
- 7,1 à 7,5 ppm (m, 3H)
- 8 ppm (m, 1H)
- 8,7 ppm (d, 1H)

*Stade b. (chloro-2 phényl)-1, (thiényl-2)-4, aza-2, butadiène-1,3-*

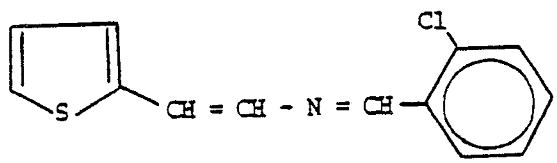

A un mélange voilemment agité d'une solution aqueuse de soude à 50% et ce 80 ml de toluène, on ajoute 1,47 g (4 mmole) d'iodure de tétra-n-butyl ammonium, puis goutte à goutte, une solution de 28,95 g (0.1 mole) de l'imine préparée précédemment et de 11,2 g (0,1 mole) de thenaldéhyde-2 dans 20 ml de toluène.

Après la fin de l'addition au cours de laquelle le température s'élève de 20 à 35°C, on porte le mélange réactionnel toujours sous agitation à 40°C pendant 30 minutes.

Après refroidissement et séparation des phases par décantation, la phase aqueuse isolée est extraite 2 fois avec 50 ml de toluène. Les phases organiques réunies sont lavées à l'eau, séchées sur sulfate de sodium puis évaporées.

On obtient 18,8 g (rendement 80%) de (chloro-2 phényl)-1 (thiényl-2)-4, aza-2, butadiène-1,3 sous forme d'un huile jaune pratiquement monopic en CPG (OV 17).

IR (film): C=N 1640 $cm^{-1}$

RMN ($CDCl_3$):
- H | C=N 8,6 ppm (s, 1H)
- 8 ppm (m, 1H)
- 8,9 à 7,9 ppm (m, 8H)

*Stade c. chlorhydrate de la N-orthochlorobenzyl, (thiényl-2)-2 éthylamine*

A une solution de 6,06 g (0,16 mole) de borohydrure de sodium dans 150 ml d'éthanol, on ajoute goutte à goutte en 5 minutes l'azadiène brut précédent (19,8 g) en solution dans 50 ml d'éthanol.

Après la fin de l'addition, au cours de laquelle la température s'élève de 20 à 30°C, on porte lentement le milieu réactionnel à une température comprise entre 45 et 50°C et on laisse la réaction se poursuivre à cette température pendant une heure.

Le milieu réactionnel est ensuite évaporé et le résidu obtenu est repris par de l'éther isopropylique. La phase éthérée est lavés plusieurs fois à la soude N, à l'eau; séchée sur sulfate de sodium puis évaporée pour fournir 20 g (rendement 100%) de N-Othochlorobenzyl (thiényl-2)-2 éthylamine sous forme d'une huile jaune clair.

A la base brute obtenue et mise en suspension dans 50 ml d'eau, on ajoute goutte à goutte à 50°C 8,3 ml d'acide chlorhydrique aqueux 12 N puis on porte le mélange à 90°C. La solution homogène ainsi obtenue laisse précipiter par refroidissement des cristaux que l'on filtre, lave à l'eau permutée puis sèche à 50°C sous vide. On obtient ainsi 20,4 g (rendement 71% par rapport à l'aminométhylphosphonate de diéthyle engagé au stade a) de chlorhydrate de N-orthochlorobenzyl- (thiényl-2)£2 éthylamine sous forme de cristaux blancs.

F = 143°C

IR (pastille KBr):
- 3400 $cm^{-1}$
- 2900 à 2600 $cm^{-1}$
- 1575 $cm^{-1}$
- 1450 $cm^{-1}$

RMN (DMSO $d_6$) δ/TMS:
- 7 à 7,8 ppm (m, 8H)
- 3,35 ppm (s, 4H)
- 4,15 ppm (s, 2H)
- env. 9 ppm (m, 2H) échangeable avec $D_2O$

Analyse $C_{13}H_{14}ClNS$, HCl = 288,236

| | C% | H% | N% |
|---|---|---|---|
| Calculé: | 54,16 | 5,24 | 4,85 |
| Trouvé: | 54,07 | 5,31 | 4,80 |

Exemple 2

Preparation du chlorhydrate de la N-orthochlorobenzyl (Thienyl-3)-2 ethylamine

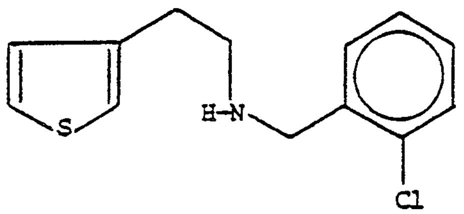 , HCl

*stade a. N-orthochlorobenzylidène, aminométhylphosphonate de diéthyle*

En opérant comme à l'exemple 1, et sur les mêmes quantités, on obtient 29 g (rendement 100%) du produit référencé.

*stade b. (chloro-2 phényl)-1 (thiényl-3)-4, aza-2, butadiène-1,3*

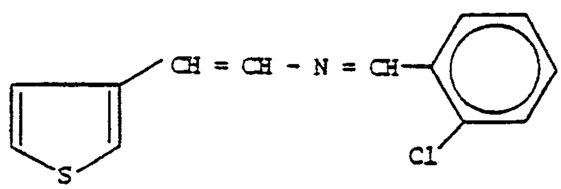

En poursuivant comme à l'exemple 1, mais en mettant en oeuvre la thénaldéhyde-3, on obtient après traitement 19,6 g (rendement 79%) d'aza-2 butadiène-1,3 sous forme d'une huile jaune que l'on met en réaction dans le stade suivant telle que.

*stade c. chlorhydrate de la N-orthochlorobenzyl, (thiényl-3)-2 éthylamine*

En opérant comme à l'exemple 1 sur le produit préparé ci-dessus, on obtient 19,2 g (rendement 66% par rapport à l'aminométhyl phosphonate de diéthyle engagé au stade a) sous forme de cristaux blancs.

F = 176°

RMN (DMSO $d_6$) δ/TMS:
- 3,2 ppm (s, 4H)
- 4,05 ppm (s, 2H)
- env. 9 ppm (m, 2H) échangeable avec $D_2O$
- 6,9 à 7,8 ppm (m, 7H)

IR (Pastilles KBr):
- 3400 $cm^{-1}$
- 2900 $cm^{-1}$
- 2700—2800 $cm^{-1}$
- 1575 $cm^{-1}$
- 1450 $cm^{-1}$

Analyse: $C_{13}H_{14}Cl$ NS, HCl = 288,236

| | C% | H% | N% |
|---|---|---|---|
| Calculé: | 54,16 | 5,24 | 4,85 |
| Trouvé: | 54,13 | 5,30 | 4,82 |

Exemple 3

Chlorhydrate de la N-(thienyl-2)methyl, (thienyl-2)-2 ethylamine

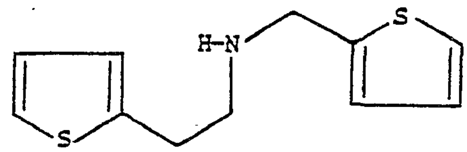

, HCl

*stade a. N-thénylidène-2, aminométhylphosphonate de diéthyle*

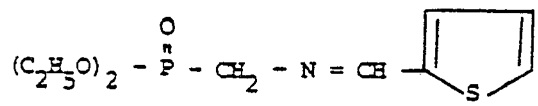

Une solution de 16,7 g (0,1 mole) d'aminométhylphosphonate de diéthyle dans 200 ml d'éthanol absolu est additionnée de 11,2 g (0,1 mole) de thenaldéhyde-2, portée au reflux puis évaporée en fournissant 26 g de N-thénylidène-2, aminométhylphosphonate de diéthyle (rendement env. 100%) sous forme d'une huile jaune monotache en CCM (plaque de silice; éluant: acétate d'éthyle).

IR (film):
- C=N 1640 $cm^{-1}$
- P=O 1260 $cm^{-1}$
- P—O—C 1060—1080 $cm^{-1}$

RMN ($CDCL_3$) δ/TMS:
- 1,35 ppm (t, 6H)
- 3,9 à 4,45 ppm (m, 6H)
- 7 à 7,8 ppm (m, 3H)
- 8,5 ppm (d, 1H)

*stade b. (thiényl-2)-1, (thiényl-2)-4, aza-2, butadiène-1,3*

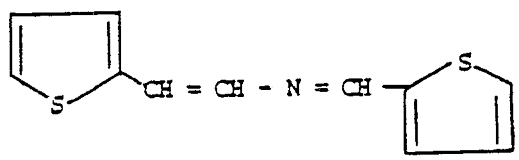

En opérant dans les conditions décrites à l'exemple 1, on obtient 18,6 g (rendement 85%) d'aza-2 butadiène-13 référencé sous forme de cristaux jaunes.

F = 163°C
IR (pastille KBr): C=N 1635 $cm^{-1}$
RMN (DMSO $d_6$):
- 8,35 ppm (s, 1H)
- 6,9 à 7,5 ppm (m, 8H)

Analyse: $C_{11}H_9NS_2$ = 219,322

| | | | |
|---|---|---|---|
| Calculé: | C% 60,27 | H% 4,10 | N% 6,39 |
| Trouvé: | 60,25 | 4,07 | 6,40 |

*stade c. chlorhydrate de la N-(thiényl-2)méthyl, (thiényl-2)-2 éthylamine*

A 10,95 g (0,05 mole) d'aza-2, diène-1,3 obtenu ci-dessus, en suspension dans 100 ml d'éthanol, on additionne peu à peu 3,7 g (0,1 mole de borohydrure de sodium) puis on porte le milieu réactionnel à 40—45°C. Après deux heures d'agitation à cette température, le milieu devenu homogène et incolore est traité comme à l'exemple 1. La base brute obtenue, mise en solution dans de l'éther isopropylique est additionnée d'une solution d'acide chlorhydrique gazeux 4,5 N dans le même solvant. le précipité formé est filtré, lavé à l'éther isopropylique puis séché à 50°C sous vide. On obtient ainsi 11,15 g (rendement 73% par rapport à l'aminométhylphosphonate de diéthyle) de chlorhydrate de N-(thiényl-2)méthyl, (thiényl-2)-2 éthylamine sous forme de cristaux blancs.

F = 230°C (déc)
IR (pastilles KBr):
- 3400 $cm^{-1}$
- 2920 $cm^{-1}$
- 2750 $cm^{-1}$
- 1440 $cm^{-1}$
- 1250 $cm^{-1}$

RMN (DMSO $D_6$)
- 6,9 à 7,5 ppm (m, 6H)
- 4,40 ppm (s, 2H)
- 3,2 ppm (m, 4H)
- 9 ppm (m, 2H) échangeables avec $D_2O$

Analyse: $C_{11}H_{13}NS_2$, HCl = 259,813

| | C% | H% | N% |
|---|---|---|---|
| Calculé: | 50,86 | 5,39 | 5,39 |
| Trouvé: | 50,90 | 5,40 | 5,37 |

Exemple 4

N-orthochlorobenzyl [terbutoxy-6 (thienyl-2)] ethylamine

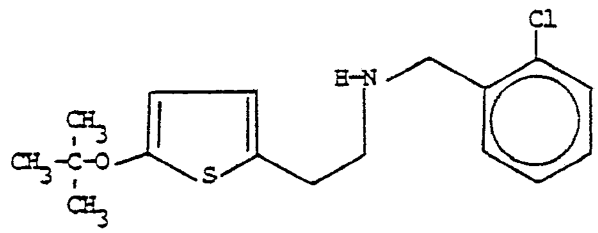

*stade a. N-orthochloro-benzylidène, aminométhylphosphonate de diéthyle*

On opère sur les mêmes quantités et dans les mêmes conditions qu'à l'exemple 1.

*stade b. (chloro-2 phényl)-1, [terbutoxy-5 (thiényl-2)]-4, aza-2, butadiène-1,3*

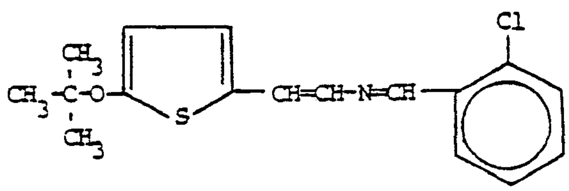

A une solution de 0,1 mole d'imine préparée ci-dessus dans 100 ml de THF* anhydre, on additionne goutte à goutte à −78°C et sous azote 35,7 ml d'une solution 2,8 M (0,1 mole de n-butyllithium dans l'hexane.

La solution devenue rouge sombre est maintenue sous agitation à −78°C et sous azote durent 30 minutes puis additionnée dans les mêmes conditions d'une solution de 18,4 g (0,1 mole) de terbutoxy-5 thénaldéhyde-2, dans 20 ml de THF* anhydre. (*THF ou tétrahydrofurane).

L'addition terminée, la solution jaune est agitée à température ambiante durant une heure puis évaporée sous vide. Le résidu après reprise par l'eau, est extrait deux fois à l'éther isopropylique. Les phases organiques réunies, séchées sur sulfate de sodium puis évaporées sous vide fournissent 32 g de (chloro-2 phényl)-1 [terbutoxy-5 (thiényl-2]-4, aza-2, butadiène-1,3 sous forme d'une huile jaune utilisée telle que dans le stade suivant.

*stade c. N-orthochlorobenzyl [terbutoxy-5 (thiényl-2)]-2 éthylamine*

A partir de 16 g (0,05 mole) de l'aza-2 butadiène-1,3-préparé ci-dessus et de 3,8 g (0,1 mole) de borohydrure de sodium et en opérant comme à l'exemple 1, on obtient 13 g de l'amine référencée sous forme d'une huile jaune qui est purifiée par l'intermédiaire de son oxalate. On obtient ainsi 8,9 g (rendement 55% par rapport à l'aminométhylphosphonate de diéthyle) de N-orthochlorobenzyl [terbutoxy-5 (thiényl-2)]-2 éthylamine sous forme d'une huile jaune clair.

F oxalate = 202°C dec
IR (film) sur la base
- 3300 $cm^{-1}$
- 2850 à 3000 $cm^{-1}$
- 1560 $cm^{-1}$
- 1150 $cm^{-1}$

RMN ($CDCl_3$) sur la base δ/TMS:
- 1,3 ppm (s, 9H) $(CH_3)_3C$
- 1,7 ppm (s, 1H) échangeable avec $D_2O$
- 2,8 ppm (s, 4H) 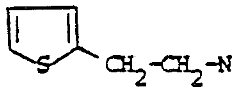
- 3,85 ppm (s, 2H) $-N-CH_2-Ar$
- 6,05 ppm (d, 1H)
- 6.35 ppm (d, 1H) 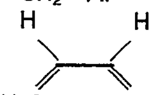 système AB avec JAB = $4H_2$
- 7,2 ppm (m, 4H)

Analyse $C_{17}H_{22}NCl\ OS$, $C_2H_2O_4$ = 413,91

| | C% | H% | N% |
|---|---|---|---|
| Calculé: | 55,13 | 5,84 | 3,39 |
| Trouvé: | 55,02 | 5,87 | 3,37 |

Exemple 5

Preparation du chlorhydrate de la N-orthochlorobenzyl, methyl-2, (thienyl-2)-2, ethylamine

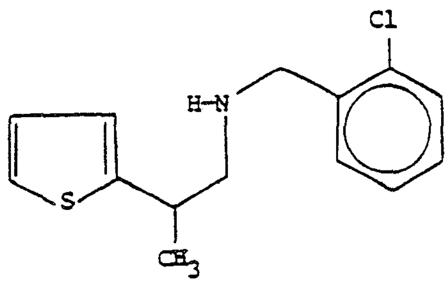
, HCl

*stade a. N-orthochlorobenzylidène, aminométhylphosphonate de diéthyle*

En opérant comme à l'exemple 1, on prépare 0,088 mole d'imine référencée.

*stade b. (chloro-2 phényl)-1, (thiényl-2)-4 méthyl-4, aza-2, butadiène-1,3*

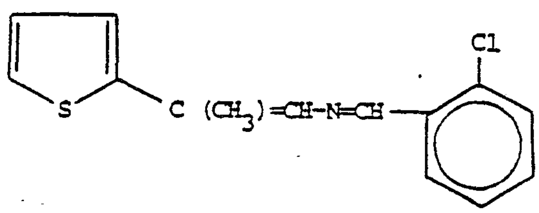

A l'imine préparée ci-dessus (0,088 mole), en solution dans 100 ml de THF anhydre, on additionne sous agitation à −78°C et sous azote 23,4 ml d'une solution à 25% (p/p) de n-butyllithium dans l'hexane (0,088 mole). A la solution rouge sombre, maintenue à −78°C, on additionne 11,1 g (0,088 mole) d'acétyl-2 thiophène dans 20 ml de THF. Le milieu réactionnel est agité deux heures à −30°C, puis 12 heures à température ambiante, versé sur une solution aqueuse saturée de chlorure d'ammonium et enfin extrait 3 fois avec 100 ml de chlorure de méthylène. Les phases organiques réunies, lavées à l'eau, séchées sur sulfate de sodium puis évaporées fournissant un résidu huileux qui se concrétise par trituration dans l'éther isopropylique pour donner 14,6 g (rendement 63%) de (chloro-2 phényl)-1, (thiényl-2)-4, méthyl-4, aza-2, butadiène-1,3 sous forme de cristaux jaunes, monotache en CCM.

F = 102°C
IR (pastille KBr):
- 3040 $cm^{-1}$
- 2940 $cm^{-1}$
- 1620 $cm^{-1}$
- 1580 $cm^{-1}$
- 1540 $cm^{-1}$
- 1465 $cm^{-1}$
- 1435 $cm^{-1}$

RMN ($CDCl_3$) δ/TMS:

- 2,2 ppm (s, 3H)
- 6,9 à 8,1 ppm (m, 8H)
- 8,75 ppm (d, 1H)

Spectre de masse:

1) *en ionisation chimique (ammoniac)*
   $(M + 1)^+ = 262—4$
2) *en impact électronique*
   $(M—Cl)^+ = 226$

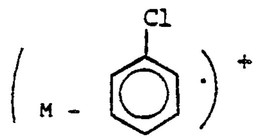
=-150

*stade c. chlorhydrate de la N-orthochlorobenzyl, méthyl-2, (thiényl-2)-2 éthylamine*

A partir de 1 g d'aza-2, butadiène-1,3 préparé ci-dessus et en opérant comme dans les exemples précédents, on obtient 0,8 g (rendement 70% par rapport à l'azadiène) de chlorhydrate référencé, sous forme de cristaux blancs.

F = 120°C

IR (pastille KBr):

- 3400 $cm^{-1}$
- 3000—2800 $cm^{-1}$
- 1570 $cm^{-1}$
- 1460 $cm^{-1}$

RMN (DMSO $d_6$) δ/TMS:

- 1,32 ppm (d, 3H)
- 3,05 ppm (d, 2H)
- 3,45 ppm (m, 1h)
- 4,1 ppm (s, 2H)
- 6,95 à 7,6 ppm (m, 7H)
- env. 9 ppm (m, 2H) échangeable avec $D_2O$

Spectre de masse:

1) *en ionisation chimique (ammoniac)*
   $M + 1)^+$ 266
   268
2) *en impact électronique*

154 - 157 = 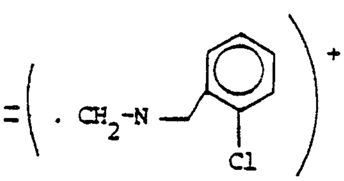

125 - 127 = 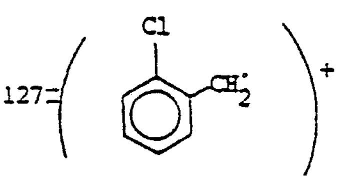

Exemple 6

Chlorhydrate de la N-orthochlorobenzyl, phenyl-1 (thienyl-2)-2 ethylamine

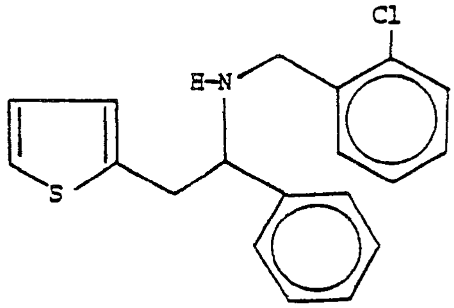

*stade a. N-orthochlorobenzylidène, alpha-phényl, aminoéthylphosphonate de diéthyle*

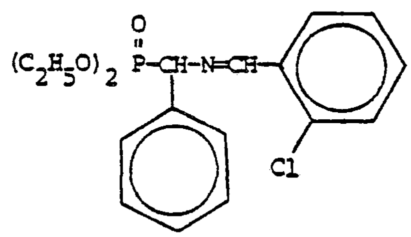

En opérant comme décrit à l'exemple 3, à partir de 12,15 g (0,05 mole) d'alpha-phényl, aminométhylphosphonate de diéthyle et 7,02 g (0,05 ole) d'orthochlorobenzaldéhyde, on obtient 18,3 g (rendement 100%) de produit référencé sous forme d'une huile jaune clair monotache en CCM.

IR (film):
- 2920 à 3100 $cm^{-1}$
- 1635 $cm^{-1}$
- 1250 $cm^{-1}$
- 1030—1050 $cm^{-1}$
- 970 $cm^{-1}$

RMN ($CDCl_3$) δ/TMS:
- 1,2 ppm (t, 6H)
- 3,9 ppm (m, 4H)
- 4,8 ppm (d, 1H)
- 7—7,8 ppm (m, 8H)
- 8,1 ppm (m, 1H)
- 8,65 ppm (d, 1H)

*stade b. (chloro-2, phényl)-1, phényl-3, (thiényl-2)-4, aza-2, butadiène-1*

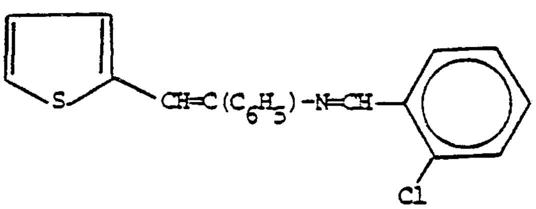

A 3,65 g (0,01 mole) d'imine préparée ci-dessus, en solution dans 50 ml de THF anhydre, on additionne sous agitation, à −78°C et sous azote, 3,6 ml (0,1 mole) d'une solution 2,8 M de n-butyllithium dans l'hexane. Après 30 minutes d'agitation supplémentaire, on additionne, toujours à −78°C, une solution de 1,12 g (0,01 mole) de thénaldéhyde-2 dans 20 ml de THF. Le milieu réactionnel est agité ensuite durant deux heures à température ambiante puis évaporé. Le résidu est repris par un mélange d'eau et de chloroforme. La phase organique isolée, lavée plusieurs fois à l'eau, séchée sur sulfate de sodium puis évaporée fournit une huile qui se concrétise par trituration dans le méthanol pour donner 1,68 g (rendement 52%) d'aza-2 butadiène-1,3 référencé sous forme de cristaux jaunes.

F = 118°C

IR (pastille KBr):

3000—3100 $cm^{-1}$
1620 $cm^{-1}$
1470—1440 $cm^{-1}$
1265 $cm^{-1}$

RMN ($CDCl_3$) δ/TMS:

8,85 ppm (s, 1H)
6,95 à 8,1 ppm (m, 13H)

Spectre de masse:

1) *en ionisation chimique (ammoniac)*
M + 1)$^+$ 324—326
2) *en impact électronique*
$M^+$: 323—325
(M—Cl)$^+$: 228

*stade c. chlorhydrate de la N-orthochlorobenzyl, phényl-1, (thiényl-2)-2 éthylamine*

A une solution de 1 g (0,003 mole) d'aza-2 butadiène-1,3 préparée ci-dessus dans 20 ml de dioxanne, on additionne sous agitation 0,57 g (0,015 mole) de borohydrure de sodium, refroidit la suspension à 0°C puis additionne à cette température goutte à goutte 1,15 ml (0,015 mole) d'acide trifluoroacétique. Le milieu réactionnel porté au reflux durant deux heures et devenu homogène est versé après refroidissement dans de l'eau puis extrait au chloroforme. La phase organique isolée, lavée plusieurs fois à l'eau, séchée sur sulfate de sodium puis évaporée, fournit une huile jaune pâle qui est chlorhydratée en milieu éthanolique pour fournir 0,58 g (rendement 52% par rapport à l'azadiène) de chlorhydrate de N-orthochlorobenzyl, phényl-1, (thiényl-2)-2 éthylamine sous forme de cristaux blancs.

F = 214°C

IR (pastille KBr):

3400 $cm^{-1}$
2950—2700 $cm^{-1}$
1565—1450 $cm^{-1}$

RMN (DMSO $d_6$):

3,1 ppm (d, 2H)
4,95 ppm (t, 1H)
4,2 ppm (s, 2H)
6,9 à 7,95 ppm (m, 12H)
env. 9 ppm (m, 2H) échangeable avec $D_2O$

Spectre de masse:

1) *en ionisation chimique (ammoniac)*
M + 1)$^+$ 328—330
2) *en impact électronique*

230-232

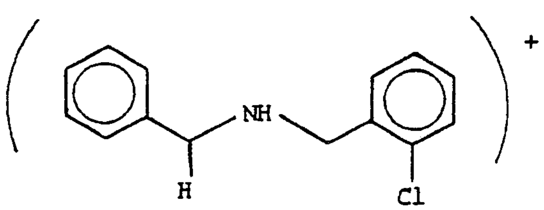

125-127

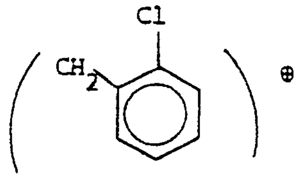

Exemple 7

Oxalate de la N-furfuryl-2, (thienyl-2)-2 ethylamine

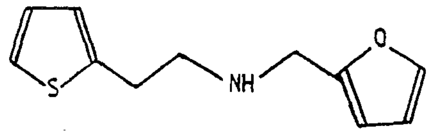 , HOOC-COOH

*Stade a. N-furfurylidène-2, aminométhylphosphonate de diéthyle*

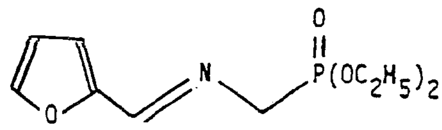

On prépare 0,1 mole de produit référencé (sous forme d'une huile jaune) en opérant comme décrit à l'exemple 1.

IR (film):

- 1645 $cm^{-1}$
- 1250 $cm^{-1}$
- 1060 $cm^{-1}$
- 1050 $cm^{-1}$

RMN ($CDCl_3$) δ/TMS:

- 1,3 ppm (t, 6H)
- 4 ppm (m, 6H)
- 7 à 7,5 ppm (m, 3H)
- 8,3 ppm (d, 1H)

*stade b. (Furyl-2)-1, (thiényl-2)-4, aza-2, butadiène 1,3*

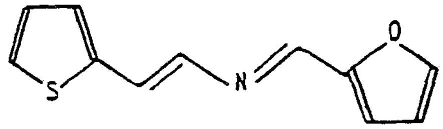

A une solution de 0,1 mole d'imine préparée ci-dessus dans 100 ml de THF anhydre on additionne goutte à goutte à −78°C et sous azote 35,7 ml d'une solution 2,8 M (0,1 mole) de n-butyllithium dans l'hexane.

La solution est maintenue sous agitation à −78°C et sous azote durant 30 minutes puis additionnée dans les mêmes conditions d'une solution de 11,2 g (0,1 mole) de thénaldéhyde-2 dans 20 l de THF anhydre.

L'addition terminée la solution est agitée à température ambiante durant 1 heure puis évaporée sous vide. Le résidue après reprise à l'eau est extrait à l'éther isopropylique. Les phases organiques réunies, séchées sur sulfate de sodium puis évaporées sus vide fournissent 22 g de (furyle-2)-1, (thiényl)-2-4, aza-2, butadiène-1,3 sous forme d'une huile jaune utilisée telle que dans le stade suivant.

*Stade c. Oxalate de la N-furfuryl-2, (thiényl-2)-2 éthylamine*

A partir de l'azadiène préparé ci-dessus et de 7,6 g (0,2 mole) de borohydrure de sodium et en opérant comme dans l'exemple 1, on prépare l'amine référencée ci-dessus (sous forme d'une huile jaune) que l'on purifie par l'intermédiaire de son oxalate.

On obtient ainsi 17,3 (rendement 58% par rapport à l'aminométhylphosphonate de diéthyle), d'oxalate de N-(furfuryl-2), (thiényl-2)-2 éthylamine sous forme de cristaux.

F = 215°C

IR (pastille KBr):

- 3400 $cm^{-1}$
- 3040 $cm^{-1}$
- 2850 $cm^{-1}$
- 1715 $cm^{-1}$
- 1650 $cm^{-1}$
- 1480 $cm^{-1}$

RMN ($CDCl_3$, sur base libérée de l'oxalate) δ/TMS:

1,65 ppm (s, 1H) échangeable avec $D_2O$

2,8 ppm (s, 4H) 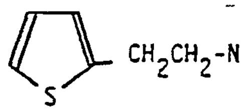

3,65 ppm (s, 2H) 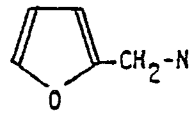

6,1 ppm (m, 2H)

6,6 à 7,3 ppm (m, 4H)

Analyse $C_{11}H_{13}NOS$, $C_2H_2O_4$ = 297,324

| | C% | H% | N% |
|---|---|---|---|
| Calculé: | 52,52 | 5,05 | 4,71 |
| Trouvé: | 52,50 | 5,03 | 4,65 |

Exemple 8

N-(picolyl-4), (thienyl-2)-2 ethylamine

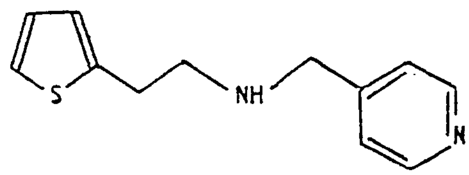

*stade a. Imine du pyridyl-4, carboxaldéhyde et de l'aminométhyl, phénylphosphinate d'isopropyle*

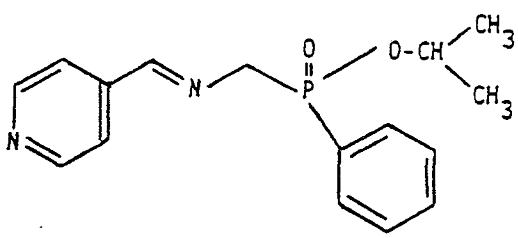

On prépare 0,1 mole du produit référencé (sous forme d'une huile jaune) en opérant comme décrit à l'exemple 1.

IR (film):

- 3000 $cm^{-1}$
- 1630 $cm^{-1}$
- 1600 $cm^{-1}$
- 1200 $cm^{-1}$
- 980 $cm^{-1}$

RMN ($CDCl_3$) δ/TMS

- 1,4 ppm (d de d, 6H)
- 4,15 ppm (d, 2H)
- 4,7 ppm (m, 1H)
- 7 à 7,8 ppm (m, 7H)
- 8,25 ppm (d, 1H)
- 8,55 ppm (d, 2H)

*stade b. (Pyridyl-4)-1, (thiényl-2)-4, Aza-2, butadiène-1,3*

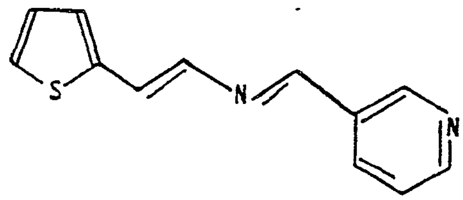

En opérant comme décrit à l'exemple 7 on isole 18,2 g (rendement 85,3%) d'azadiène de référence sous forme d'une huile orangée que l'on utilise telle quelle au stade suivant.

*stade c. N-(Picolyl-4), (Thiényl-2)-2 éthylamine*

A partir de l'azadiène préparé ci-dessus et de 7,6 g (0,2 mole de borohydrure de sodium, et en opérant comme à l'exemple 1 on prépare l'amine référencéee ci-dessus sous forme d'une huile marron que l'on purifié par chromatographie sur colonne se silice.

On obtient ainsi 9,17 g (rendement 42% par rapport à l'aminométhylphosphinate) de N-(picoly-4), (thiényl-2)-2 éthylamine sous forme d'une huile légèrement colorée.

IR (film):
- 3300 $cm^{-1}$
- 2900 $cm^{-1}$
- 1600 $cm^{-1}$
- 1440 $cm^{-1}$

RMN ($CDCl_3$) δ/TMS:
- 1,7 ppm (s, 1H) échangeable avec $D_2O$
- 3 ppm (t, 4H) Ar—$CH_2$—$CH_2$
- 3,8 ppm (s, 2H) Ar—$CH_2$—N
- 6,6 à 7,4 ppm (m, 5H)
- 8,4 ppm (d, 2H)

## Exemple 9

### Chlorhydrate de la N-orthonitrobenzyl, (thienyl-2)-2 ethylamine

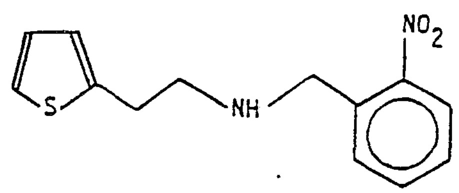

, HCl

*stade a. N-orthonitrobenzylidène, aminométhyl, phénylphosphinate d'isopropyle*

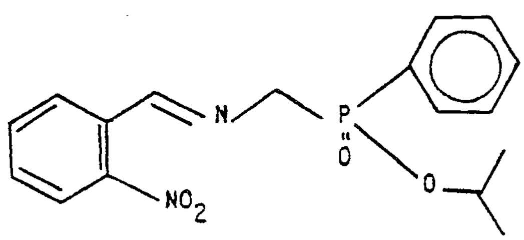

A partir de 0,1 mole d'orthonitrobenzaldéhyde et de 0,1 mole d'aminométhyl, phénylphosphinate d'isopropyle on obtient (en opérant comme décrit à l'exemple 1) 0,1 mole (100%) de l'imine référencée sous forme d'une huile jaune.

IR (film):
- 1630 $cm^{-1}$
- 1200 $cm^{-1}$
- 1980 $cm^{-1}$

RMN ($CDCl_3$):
- 1,5 ppm (d de d, 6H)
- 4,25 ppm (d, 1H)
- 7,5 à 8,3 ppm (m, 9H)
- 8,6 ppm (d, 1H)

*stade b. Orthonitrophényl-1, (thiényl-2)-4, aza-2, butadiène-1,3*

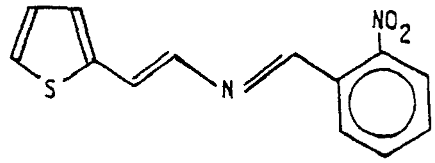

A partir de 0,1 mole dd'imine préparée ci-dessus et de 0,1 mole de thénaldéhyde-2 et en opérant comme décrit à l'exemple 3 on obtient 22 g d'azadiène référencé (rendement 85% par rapport à l'amino-méhylphosphinate) sous forme d'une huile que l'on utilise telle quelle à l'étape suivante.

*stade c. Chlorhydrate de N-orthonitrobenzyl, (thiényl-2)-2 éthylamine*

A l'azadiène obtenu ci-dessus, en solutin dans 200 ml d'éthanol, sont additionnés par petites portions 6,46 g (0,17 mole) de borohydrure de sodium, en maintenant la température inférieure à 25°C. Le milieu réactionnel est ensuite agité 2 heures à température ambiante puis versé dans 1 l d'eau et extrait au chloroforme.

La phase organique lavée à l'eau, séchée sur sulfate de sodium puis évaporée fournit la base sous forme d'une huile qui est chlorhydratée dans l'éthanol. Après recristallisation du précipité formé dans l'éthanol, on obtient 18,2 g (rendement 61% par rapport à l'aminométhylphosphinate) de chlorhydrate de N-orthonitrobenzyl, (thiényl-2)-2 éthylamine sous forme de cristaux blancs.

F = 168°C

IR (pastille KBr):
- $3450\ cm^{-1}$
- $3000\ cm^{-1}$
- $2900\ cm^{-1}$
- $2700\ cm^{-1}$
- $1560\ cm^{-1}$
- $1525\ cm^{-1}$
- $1450\ cm^{-1}$
- $1340\ cm^{-1}$

RMN ($CDCl_3$ sur la base libérée du chlorhydrate) δ/TMS:
- 1,65 ppm (s, 1H) échangeable avec $D_2O$
- 2,9 ppm (t, 4H)
- 4 ppm (s, 2H)
- 6,7 à 7,9 ppm (m, 7H)

Analyse $C_{13}H_{14}N_2O_2S$, HCl: 298,773

| | C% | H% | N% |
|---|---|---|---|
| Calculé: | 52,26 | 5,06 | 9,38 |
| Trouvé: | 52,28 | 5,03 | 9,31 |

Exemple 10

Chlorhydrate de la N-orthochlorobenzyl, [thiényl-2]-2 éthylamine

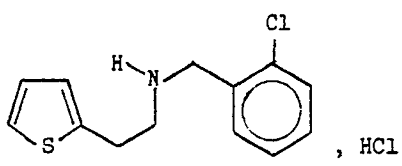

*Stade a): Oxyde de N-orthochlorobenzylidène, aminométhyl, diphénylphosphine*

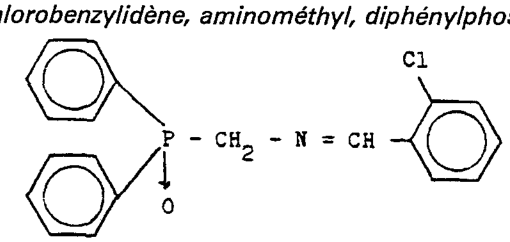

A une solution de 3,5 g (0,015 mole) d'oxyde d'aminométhyl, diphénylphosphine dans 150 ml d'éthanol, on additionne, à température ambiante, goutte à goutte 2,18 g (0,015 mole) d'orthochlorobenz-aldéhyde. Après évaporation du solvant par chauffage à 50°C sous léger vide, on obtient une huile jaune clair qui est reprise à chaud par 50 ml d'un mélange 25/1 d'éther isopropylique et d'éthanol pour donner après refroidissement à +10°C, filtration et séchage à 40°C sous vide, 4,9 g de cristaux du produit attendu. F = 89°C.

*Stade b): (Chloro-2 phényl)-1, (thiényl-2)-4, aza-2 butadiène-1,3*

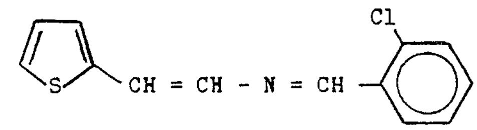

A un mélange violemment agité de 10 g d'une solution aqueuse de soude à 50% et de 0,18 g d'iodure de tétra-n-butylammonium, on ajoute, goutte à goutte, une solution de 2,65 g (0,0075 mole) de l'imine préparée ci-dessus et de 0,84 g (0,0075 mole) de thénaldéhyde-2 dans 8 ml de toluène. L'agitation est ensuite maintenue à température ambiante durant une heure. La phase toluénique isolée après décantation, est lavée avec 50 ml d'une solutin aqueuse à 2% de chlorure de sodium puis évaporée sous vide. L'huile résiduelle obtenue, après reprise par 6 ml d'éthanol et refroidiseement, se transforme en cristaux qui sont filtrés et séchés sous vide à température ambiante. On obtient ainsi 1,05 g de (chloro-2 phényl-1)-1 (thiényl-2)-4, aza-2, butadiéne-1,3. F = 65°C.

*Stade c): Chlorhydrate de la N-orthochlorobenzyl, (thiényl-2)-2 éthylamine*

En opérant tel que décrit à l'exemple 1, à partir de 0,45 g de l'azadiène obtenu ci-dessus et de 0,2 g de borohydrure de sodium, on obtient 0,4 g de produit attendu. F = 143°C.

**Revendications**

1. Procédé de préparation de dérivés de (thiényl-2)- et (thiényl-3)-2-éthylamines de formule

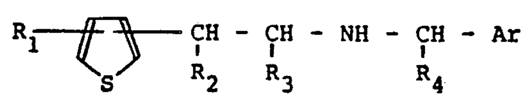

(I)

dans laquelle,

$R_1$, en position 2, 3, 4 ou 5 représente un atome d'hydrogène, d'halogène, un radical nitro, carboxyle, cyano, amino, alkyle ou alcoxy linéaire ou ramifié,

dans la chaine amino éthyle qui peut occuper les positions 2 ou 3, $R_2$, $R_3$ et $R_4$ représentent un atome d'hydrogène ou un radial alkyle ou phényle,

Ar représente un radical thiényle, furyle, pyridyle ou phényle, éventuellement mono- ou poly-substitué par un atome d'halogène ou un groupe nitro, caractérisé en ce qu'on condense un dérivé de formule

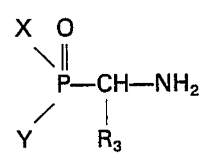

(II)

dans laquelle $R_3$ a les significations précitées, X et Y sont identiques ou différents et représentent un radical alcoxy ou phényle, avec un composé carbonylé de formule

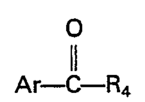

(III)

dans laquelle Ar et $R_4$ ont les significations précitées, obtenant ainsi un dérivé de formule

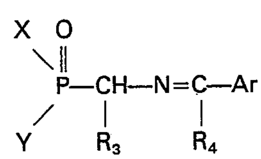

(IV)

dans laquelle X, Y, Ar, $R_3$ et $R_4$ ont les significations précitées, que l'on traité par une base de formule $B^{\ominus} M^{\oplus}$ pour obtenir un carbanion de formule

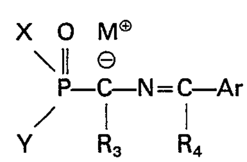

(V)

que l'on met à réagir avec un dérivé carbonylé de formule

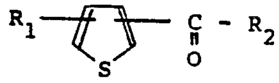

(VII)

dans lequel $R_1$ et $R_2$ ont les valeurs précitées, pour obtenir le composé de formule

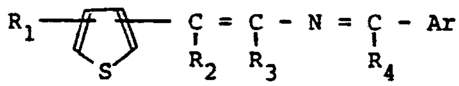

(VIII)

dans lequel $R_1$, $R_2$, $R_3$, $R_4$ et Ar ont les valeurs précitées que l'on traite par un agent réducteur pour obtenir le composé de formule (I).

2. Procédé suivant la revendication 1, caractérisé en ce que la réaction de condensation entre le composé organophosphoré (II) et le composé carbonylé (III) dans le cas où ce dernier est une cétone ($R_4 \neq H$), est catalysée par la présence d'un acide minéral ou organique tel que l'acide para-toluène sulfonique.

3. Procédé suivant une des revendications 1 ou 2, caractérisé en ce que la formation du carbanion en présence de la base s'effectue à des températures comprises entre −78°C et +100°C.

4. Procédé selon la revendication 3, caractérisé en ce que $M^{\oplus}$ est un métal alcalin ou alcalino-terreux et $B^{\ominus}$ est un carbanion choisi parmi les groupes chargé négativement correspondants, hydrogène, hydroxy, amino, mono- ou dialkylamino, alkyle linéaire ou ramifié, alkoxy linéaire ou ramifié ou la base $B^{\ominus}$ $M^{\oplus}$ est un composé organomagnésien ou un composé organosodé.

5. Procédé selon la revendication 4, caractérisé en ce que $M^{\oplus}$ est un métal choisi parmi le sodium, le lithium, le potassium et le magnésium.

6. Procédé suivant la revendication 4, caractérisé en ce que la base utilisée est un hydrure de métal alcalin ou alcalino-terreux.

7. Procédé suivant l'une quelconque des revendications 3 à 6, caractérisé en ce que la réaction s'effectue dans un solvant organique tel que des éthers linéaires ou cycliques, des hydrocarbures aromatiques, des alcools, des amides ou des sulfoxydes.

8. Procédé suivant la revendication 7, caractérisé en ce que la réaction s'effectue dans un solvant organique tel que le tétrahydrofuranne, le benzène, le diméthylsulfoxyde.

9. Procédé suivant une des revendications 1 à 8, caractérisé en ce que la réaction entre le carbanion (V) et le dérivé carbonylé (VII) s'effectue à des températures comprises entre −78°C et +100°C.

10. Procédé suivant une des revendications 1 à 3, caractérisé en ce que la réaction d'hydrogénation du composé III est effectuée par un borohydrure de métal alcalin tel que le borohydrure de sodium ou le borohydrure de potassium.

11. Procédé suivant la revendication 10, caractérisé en ce que la réaction s'effectue dans un solvant organique tel que le tétrahydrofuranne, le dioxane, le méthanol ou l'éthanol.

**Patentansprüche**

1. Verfahren zur Herstellung von Derivaten von 2-(2-Thienyl)- und 2-(3-Thienyl)-ethylaminen der Formel

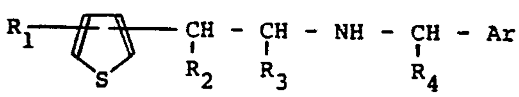

(I)

worin $R_1$ in 2-, 3-, 4- oder 5-Stellung ein Wasserstoffatom, Halogenatom, eine Nitro-, Carboxyl-, Cyano-, Aminogruppe, ein linearer oder verzweigter Alkyl- oder Alkoxyrest ist, $R_2$, $R_3$ und $R_4$ in der Aminoethylkette, die die 2- oder 3-Stellung einnehmen kann, ein Wasserstoffatom oder ein Alkylrest oder Phenylrest sind, Ar ein gegebenenfalls einfach oder mehrfach durch ein Halogenatom oder eine Nitrogruppe substituierter Thienyl-, Furyl-, Pyridyl- oder Phenylrest ist, dadurch gekennzeichnet, daß man ein Derivat der Formel

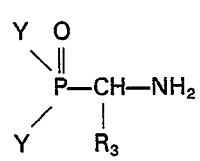

(II)

worin $R_3$ die vorstehend genannten Bedeutungen hat, X und Y gleich oder verschieden sind und einen Alkoxy- oder Phenylrest darstellen, mit einer Carbonylverbindung der Formel

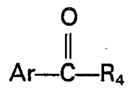

(III)

in der Ar und $R_4$ die vorstehend genannten Bedeutungen haben, kondensiert und hierbei ein Derivat der Formel

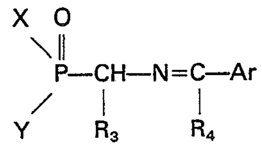

(IV)

erhalt, worin X, Y, Ar, $R_3$ und $R_4$ die vorstehend genannten Bedeutungen haben, das man mit einer Base der Formel $B^{\ominus} M^{\oplus}$ behandelt, um ein Carbanion der Formel

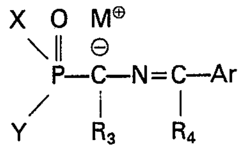

(V)

zu erhalten, das man mit einem Carbonylderivat der Formel

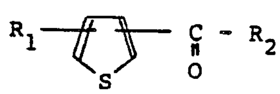

(VII)

in der $R_1$ und $R_2$ die vorstehend genannten Bedeutungen haben, umsetzt, um die Verbindung der Formel

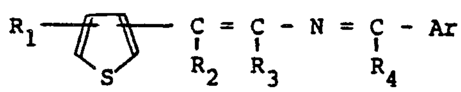

(VIII)

in der $R_1$, $R_2$, $R_3$, $R_4$ und Ar die vorstehend genannten Bedeutungen haben, zu erhalten, die man mit einem Reduktionsmittel behandelt, um die Verbindung der Formel (I) zu erhalten.

2. Verfahren nach Anspruch 1, dadurch gekennzeichnet, daß die Kondensationsreaktion zwischen der Organophosphorverbindung (II) und der Carbonylverbindung (III) in dem Fall, in dem die letztere ein Keton ($R_4 \neq H$) ist, durch die Anwesenheit einer anorganischen oder organischen Säure, z.B. p-Toluolsulfonsäure, katalysiert wird.

3. Verfahren nach einem der Ansprüche 1 oder 2, dadurch gekennzeichnet, daß die Bildung des Carbanions in Gegenwart der Base bei Temperaturen zwischen −78°C und +100°C erfolgt.

4. Verfahren nach Anspruch 3, dadurch gekennzeichnet, daß $M^{\oplus}$ ein Alkalimetall oder Erdalkalimetall und $B^{\ominus}$ ein Carbanion, das aus entsprechenden negativ geladenen Gruppen ausgewählt ist, Wasserstoff, Hydroxy, Amino, Mono- oder Dialkylamino, lineares oder verzweigtes Alkyl, lineares oder verzweigtes Alkoxy ist oder die Base $B^{\ominus} M^{\oplus}$ eine Organomagnesium- oder Organonatriumverbindung ist.

5. Verfahren nach Anspruch 4, dadurch gekennzeichnet, daß $M^{\oplus}$ ein aus Natrium, Lithium, Kalium und Magnesium ausgewähltes Metall ist.

6. Verfahren nach Anspruch 4, dadurch gekennzeichnet, daß die verwendete Base ein Alkali- oder Erdalkalimetallhydrid ist.

7. Verfahren nach irgendeinem der Ansprüche 3 bis 6, dadurch gekennzeichnet, daß die Reaktion in einem organischen Lösungsmittel, z.B. linearen oder cyclischen Ethern, aromatischen Kohlenwasserstoffen, Alkoholen, Amiden oder Sulfoxiden, durchgeführt wird.

8. Verfahren nach Anspruch 7, dadurch gekennzeichnet, daß die Reaktion in einem organischen Lösungsmittel, wie Tetrahydrofuran, Benzol und Dimethylsulfoxid, durchgeführt wird.

9. Verfahren nach einem der Ansprüche 1 bis 8, dadurch gekennzeichnet, daß die Reaktion zwischen dem Carbanion (V) und dem Carbonylderivat (VII) bei Temperaturen zwischen −78°C und +100°C durchgeführt wird.

10. Verfahren nach einem der Ansprüche 1 bis 3, dadurch gekennzeichnet, daß die Hydrierreaktion der Verbindung III mit einem Alkaliborhydrid, wie Natrium- oder Kaliumborhydrid, durchgeführt wird.

11. Verfahren nach Anspruch 10, dadurch gekennzeichnet, daß die Reaktion in einem organischen Lösungsmittel, wie Tetrahydrofuran, Dioxan, Methanol oder Ethanol, durchgeführt wird.

**Claims**

1. Process for the preparation of 2-(2-thienyl)- and -(3-thienyl)ethylamines of the formula

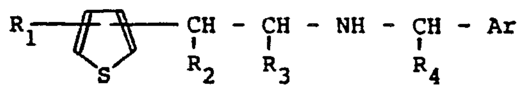

(I)

in which:

$R_1$, at 2-, 3-, 4- or 5-position represents a hydrogen atom, a halogen atom, a nitro, carboxy, cyano, amino or straight- or branched-chain alkyl or alkoxy radical

in the aminoethyl chain which may be linked to the 2- or 3-position, $R_2$, $R_3$ and $R_4$ represent a hydrogen atom or an alkyl or phenyl group,

Ar represents a thienyl, furyl, pyridyl or phenyl radical optionally mono- or poly-substituted with a halogen atom or nitro group,

comprising condensing a derivative of the formula

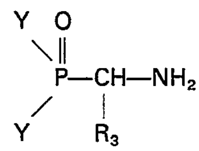

(II)

in which $R_3$ has the above-defined meaning, X and Y represent independently from each other an alkoxy, or phenyl radical, with a carbonyl compound of the formula:

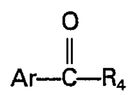

(III)

in which Ar and $R_4$ have the aforesaid meanings, to give a derivative of the formula:

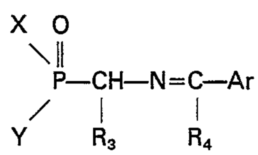

(IV)

in which X, Y, Ar, $R_3$ and $R_4$ have the aforesaid meanings;

treating compound (IV) with a base of the formula $B^{\ominus} M^{\oplus}$, to give a carbanion of the formula:

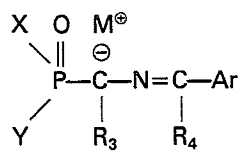

(V);

reacting the carbanion of the formula (V) with a carbonyl derivative of the formula:

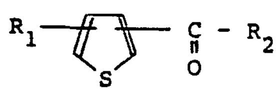

(VII)

in which $R_1$ and $R_2$ are as defined above, to give a compound of the formula:

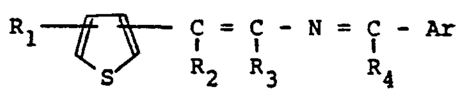

(VIII)

in which $R_1$, $R_2$, $R_3$, $R_4$ and Ar are as defined above; and

treating compound (VIII) with a reducing agent, to give a compound of the formula (I).

2. Process as claimed in claim 1, wherein the condensation reaction between organophosphorus compound (II) and carbonyl compound (III) in the case when the latter is a ketone ($R_4 = H$), is catalyzed by the presence of an inorganic or organic acid such as paratoluene sulfonic acid.

3. Process as claimed in any one of the claims 1 or 2, wherein the formation of the carbanion in the presence of said base is effected at temperatures comprised between −78°C and +100°C.

4. Process as claimed in claim 3, wherein $M^{\oplus}$ is an alkali or alkaline earth metal and $B^{\ominus}$ is a carbanion selected from the negatively charged group corresponding to hydrogen, hydroxy, amino, mono- or dialkylamino, straight- or branched chain alkyl or straight- or branched chain alkoxy or the base $B^{\ominus}$ $M^{\oplus}$ is an organomagnesium compound or an organosodium compound.

5. Process as claimed in claim 4, wherein $M^{\oplus}$ is a metal selected from sodium, lithium, potassium and magnesium.

6. Process as claimed in claim 4, wherein the base used is selected from an alkali metal hydride, an organomagnesium compound, and alkali metal and alkaline earth metal alkoxides and hydroxides.

7. Process as claimed in any one of the claims 3 to 6, wherein the reaction is effected within an organic solvent selected from straight-chain and cyclic ethers, aromatic hydrocarbons, alcohols, amides and sulfoxides.

8. Process as claimed in claim 7, wherein the reaction is effected within an organic solvent selected from tetrahydrofuran, benzene and dimethylsulfoxide.

9. Process as claimed in any one of the claims 1 to 8, wherein the reaction between carbanion (V) and carbonyl derivative (VII) is effected at temperatures comprised between −78°C and +100°C.

10. Process as claimed in any one of claims 1—11, wherein the hydrogenation reaction of compound (III) is effected with an alkali metal borohydride such as sodium borohydride or potassium borohydride.

11. Process as claimed in claim 12, wherein the reaction is effected within an organic solvent such as tetrahydrofuran, dioxan, methanol or ethanol.